Europäisches Patentamt

European Patent Office

Office européen des brevets

(19)

(11) Veröffentlichungsnummer : **0 364 664 B1**

(12)

# EUROPÄISCHE PATENTSCHRIFT

(45) Veröffentlichungstag der Patentschrift :
09.10.91 Patentblatt 91/41

(51) Int. Cl.$^5$ : **B01J 4/00**, B01J 8/02,
// C07C29/15

(21) Anmeldenummer : 89110437.4

(22) Anmeldetag : 09.06.89

(54) **Reaktor zur Durchführung katalytischer Gasreaktionen mit einer Mehrzahl von Kaltgaszuführungen.**

(30) Priorität : 29.08.88 DE 3829214

(43) Veröffentlichungstag der Anmeldung :
25.04.90 Patentblatt 90/17

(45) Bekanntmachung des Hinweises auf die
Patenterteilung :
09.10.91 Patentblatt 91/41

(84) Benannte Vertragsstaaten :
BE CH DE ES FR GB IT LI NL

(56) Entgegenhaltungen :
DE-A- 2 504 343
DE-A-15 422 09
GB-A- 2 065 492
US-A- 3 392 966
US-A- 3 556 736

(73) Patentinhaber : Uhde GmbH
Friedrich-Uhde-Strasse 15
W-4600 Dortmund 1 (DE)

(72) Erfinder : Bähnisch, Hans-Joachim
Wembersweg 30
W-4600 Dortmund 76 (DE)

(74) Vertreter : Patentanwälte Meinke und
Dabringhaus Dipl.-Ing. J. Meinke Dipl.-Ing. W.
Dabringhaus
Westenhellweg 67
W-4600 Dortmund 1 (DE)

EP 0 364 664 B1

## Beschreibung

Die Erfindung betrifft einen Reaktor zur Durchführung katalytischer Gasreaktionen mit einem Gaseinlaßstutzen im oberen Kugelboden des Reaktors für das gesamte Reaktionsgas mit einer Mehrzahl von über der Höhe des Reaktors angeordneten, in das Katalysatorbett mündenden Kaltgaszuführungen.

Aus der DE-B 1542209 ist ein derartiger Reaktor bekannt, bei dem die Kaltgaszuführungen aus quer zur Strömungsrichtung des Reaktionsgases angeordneten, Mischräume bildenden Käfigen mit perforierten Wänden bestehen, die vorzugsweise im Inneren angeordnete Rohre für die Zuleitung des Kaltgases aufweisen, wobei zwecks Ermöglichung des Eintritts eines wesentlichen Anteils des Reaktionsgases in die Käfige, diese entsprechend groß gestaltet sind. Nachteilig bei dieser bekannten Ausführungsform ist zum einen der nicht unerhebliche Raumbedarf der Käfige, wodurch der mit Katalysator gefüllte Reaktionsraum unnötig begrenzt ist. Zum anderen hat es sich gezeigt, daß keine befriedigende Vermischung der heißen Reaktionsgase mit den zugeführten Kaltgasen stattfindet, d.h., daß sich ein gewünschtes, möglichst gleichmäßiges stetiges Temperaturprofil über der Höhe des Reaktors und ein daraus resultierender hoher Reaktionsumsatzgrad nicht einstellt.

Auch in der DE-A 2504943 wird ein Reaktor zur Durchführung katalytischer Reaktionen beschrieben, der sich vom gattungsbildenden Reaktortyp jedoch dadurch unterscheidet, daß das Reaktionsgas nicht als Ganzes in einen Gaseinlaßstutzen in einem oberen Bereich des Reaktors, sondern über örtliche Teilgasströme zugeführt wird, daß das Katalysatorsystem vorzugsweise mehrzonig ausgebildet ist und daß zusätzlich mehrere, platzbedürftige und kostenerzeugende Wärmetauscher vorgesehen sind. Zum Stand der Technik ist auch ein Flüssigkeitsverteilersystem nach der US-A-3392966 zu zählen, zur Befeuchtung von Gasen.

Die Aufgabe der Erfindung besteht in der Schaffung einer Lösung, welche bei möglichst geringem Raumbedarf eine befriedigende Vermischung der zugeführten Kaltgase mit den heißen Reaktionsgasen ermöglicht, um über der Höhe des Reaktors ein möglichst gleichmäßiges, stetiges Temperaturprofil einzustellen.

Diese Aufgabe wird mit einem Reaktor der eingangs bezeichneten Art erfindungsgemäß dadurch gelöst, daß die Kaltgaszuführungen als senkrecht angeordnete, das Katalysatorbett nur bereichsweise durchdringende Düsenrohre ausgebildet sind, welche mit waagerechten Zuleitungen verbunden sind, wobei die Düsenrohre hängend an den Zuleitungen angeordnet sind, wobei die Düsenrohre jeweils mit einer Abdeckung an ihren stirnseitigen Enden verschlossen sind und wobei die Düsenrohre mit in den Seitenflächen ausgebildeten Disenöffnungen zum Austritt des eingeleiteten Kaltgases versehen sind.

Die vorliegende Erfindung hat vertikale, gleichlange Rohre, die seitliche Gasaustrittsöffnungen besitzen und am Ende geschlossen sind. Damit wird parallel zum Reaktionsgasstrom über eine längere Strecke Kaltgas zugegeben, was zu einer guten Gasmischung und Temperaturbeeinflussung führt. Diese Gasvermischung bzw. die Verdünnung des reagierenden Gases durch frisches, nicht reagierendes Gas führt zu einer hohen Reaktionsgeschwindigkeit. Durch die günstige Temperaturregelung verschiebt sich das Gleichgewicht zu Gunsten der Bildung des gewünschten Reaktionsproduktes.

In vorteilhafter Ausgestaltung der Erfindung ist vorgesehen, daß die Zuleitungen jeweils einer Ebene von je einem waagerechten, den Reaktormantel durchdringenden Zentralrohr und beiderseits desselben angeordneten, waagerechten Verteilerrohren gebildet sind. Diese Ausbildung ist einerseits konstruktiv relativ einfach herzustellen, andererseits ist durch diese Ausbildung gewährleistet, daß der für den Katalysator zur Verfügung stehende Raum ausreichend groß ist.

Dabei sind die Verteilerrohre vorteilhaft in einem einen Katalysatordurchfluß ermöglichenden Abstand nebeneinander am Zentralrohr angeordnet, wodurch eine einwandfreie Befüllung des Reaktors mit Katalysator bzw. Entleerung möglich ist, da der feinkörnige Katalysator durchrutscht.

Die Stabilität der Reaktoreinbauten kann dadurch erhöht werden, daß die Verteilerrohre mit Verstärkungsrippen versehen sind.

Schließlich sieht die Erfindung vor, daß die senkrechten Düsenrohre mit einer perforierten Ummantelung versehen sind, die vorteilhaft aus Streckmetall gebildet sein kann. Durch diese Ummantelung ist gewährleistet, daß die Düsenöffnungen der Düsenrohre sich nicht mit Katalysator zusetzen und verstopfen können, wobei andererseits die Kaltgase noch einwandfrei in das Katalysatorbett einströmen können.

Die Erfindung ist nachstehend anhand der Zeichnung beispielsweise näher erläutert. Diese zeigt in :

Fig. 1    in vereinfachter Darstellung einen Längsschnitt durch einen erfindungssgemäß ausgebildeten Reaktor,

Fig. 2    einen Querschnitt durch einen Reaktor,

Fig. 3    einen Schnitt gemäß Linie III-III in Fig. 2,

Fig. 4    ein vergrößert dargestelltes einzelnes Düsenrohr in einer Seitenansicht und

Fig. 5    einen Schnitt gemäß der Linie V-V in Fig. 4.

2

Ein allgemein mit 1 bezeichneter Reaktor ist in der Zeichnung stark vereinfacht nur mit seinen für die Erfindung wesentlichen Merkmalen dargestellt. Der Reaktor ist für eine Vielzahl von katalytischen Gasreaktionen, insbesondere aber für exotherme Reaktionen geeignet, wobei ganz bevorzugt ein Einsatz bei der Methanol-Synthese unter hohen Drücken und Temperaturen von etwa 200 bis 300°C vorgesehen ist.

Der Reaktor 1 weist einen zylindrischen Mantel 2, einen oberen Kugelboden 3 mit Gaseinlaßstutzen 4 sowie einen unteren Kugelboden 5 mit Gasauslaßstutzen 6 auf. Wenigstens im Bereich des zylindrischen Mantels 2 ist der Reaktor 1 mit einem Katalysatorbett 7 gefüllt, das sich aber auch bis in den Bereich des unteren Kugelbodens 5 erstrecken kann. Zum Befüllen des Reaktors 1 mit Katalysator bzw. zum Entleeren sind im oberen Kugelboden 3 und im unteren Kugelboden 5 entsprechende, nicht dargestellte Öffnungen vorgesehen.

Über der Höhe des Reaktors 1 im Bereich des zylindrischen Mantels 2 sind eine Mehrzahl von Kaltgaszuführungen 8 angeordnet, welche aus den zylindrischen Mantel 2 durchdringenden waagerechten Zuleitungen 9 mit daran hängend angeordneten senkrechten Düsenrohren 10 bestehen.

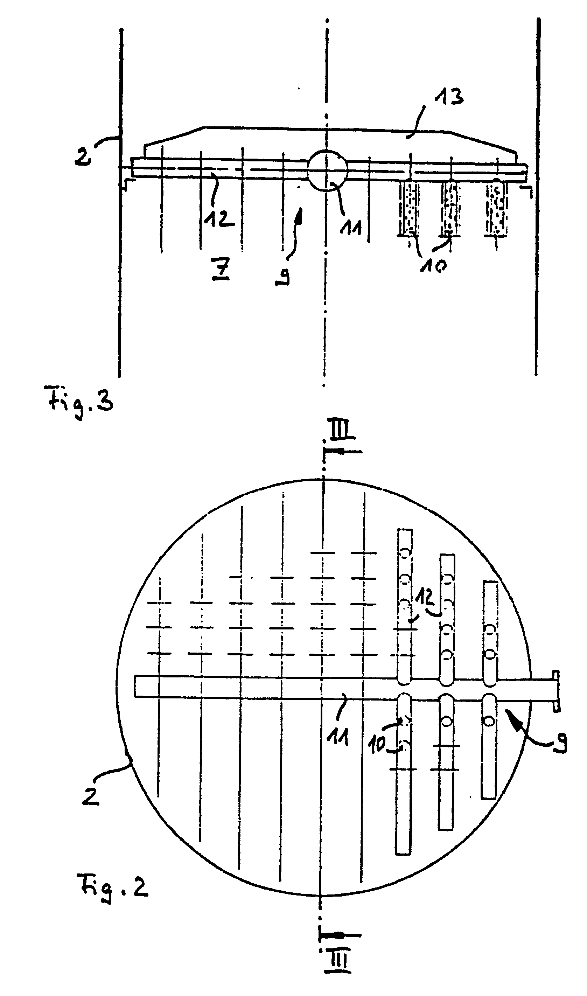

Die Ausbildung der Kaltgaszuführungen 8 ist in den Fig. 2 und 3 näher dargestellt. Die waagerechten Zuleitungen 9 jeweils einer Ebene sind von einem waagerechten, den Reaktormantel 2 durchdringenden Zentralrohr 11 mit beidseitig daran angeordneten waagerechten Verteilerrohren 12 gebildet, wobei die Länge der Verteilerrohre 12 zur Reaktormitte hin zunehmend derart ausgebildet ist, daß der gesamte Reaktorquerschnitt mit Verteilerrohren 12 ausgerüstet ist. Die Verteilerrohre 12 können vorzugsweise Verstärkungsrippen 13 aufweisen, die entsprechend mit dem Zentralrohr 11 verbunden sind.

Die Verteilerrohre 12 sind derart nebeneinander am Zentralrohr 11 angeordnet, daß der lichte Abstand zwischen den einzelnen Rohren 12 ausreichend groß ist, um einen einwandfreien Durchfluß des Katalysators beim Füllen oder Entleeren zu gewährleisten. Als besonders günstig hat sich dabei ein lichter Abstand von etwa 80 mm herausgestellt. Selbstverständlich ist dieser Abstand aber auch von der Korngröße des eingesetzten Katalysators abhängig.

Wie besonders gut aus Fig. 3 hervorgeht, sind die Düsenrohre 10 jeweils hintereinander senkrecht hängend an den Verteilerrohren 12 angeordnet. Das Zentralrohr 11 ist bevorzugt auf einem in der Innenseite des zylindrischen Mantels 2 ausgebildeten Auflagering angeordnet, wodurch ein besonders einfacher Ein- und Ausbau eines jeweiligen Zentralrohres 11 mit den Verteilerrohren 12 und Düsenrohren 10 möglich ist.

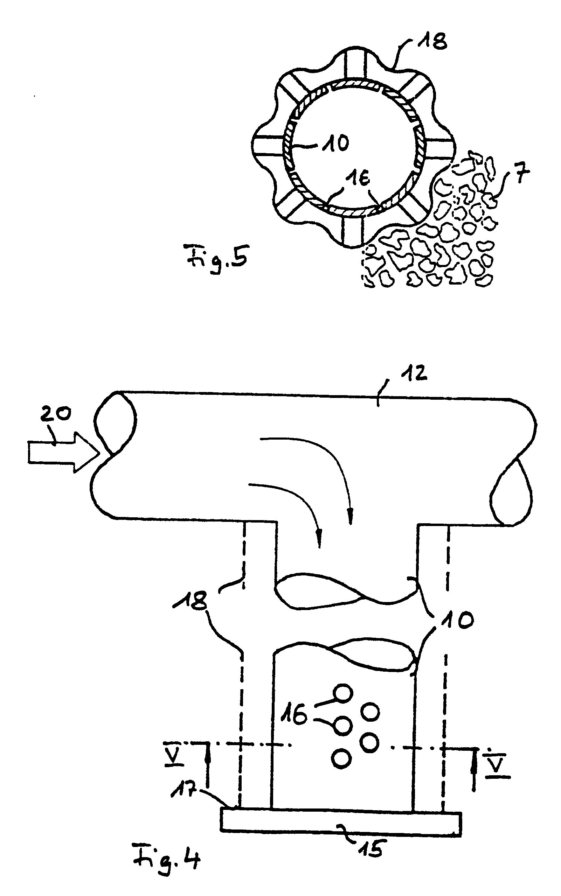

Die Ausgestaltung der Düsenrohre 10 ist in den Fig. 4 und 5 näher dargestellt. Die Düsenrohre 10 sind an ihren stirnseitigen Enden mit einer Abdeckung 15 verschlossen, so daß eingeleitetes Kaltgas nur durch in den Seitenflächen ausgebildete Düsenöffnungen 16 austreten kann. Die Abdeckung 15 ist dabei scheibenförmig mit einem größeren Außendurchmesser als das zugehörige Düsenrohr 10 ausgebildet, so daß eine ringförmige Auflagefläche 17 für eine perforierte Ummantelung 18, insbesondere aus Streckmetall ausgebildet ist. Diese Ummantelung 18 umhüllt dabei die gesamte Außenfläche des Düsenrohres 10 und verhindert, daß Katalysator in den Bereich der Düsenöffnungen 16 gelangen und diese verstopfen kann. Die Perforierung ist so gewählt, daß das austretende Kaltgas ungestört ausströmen kann.

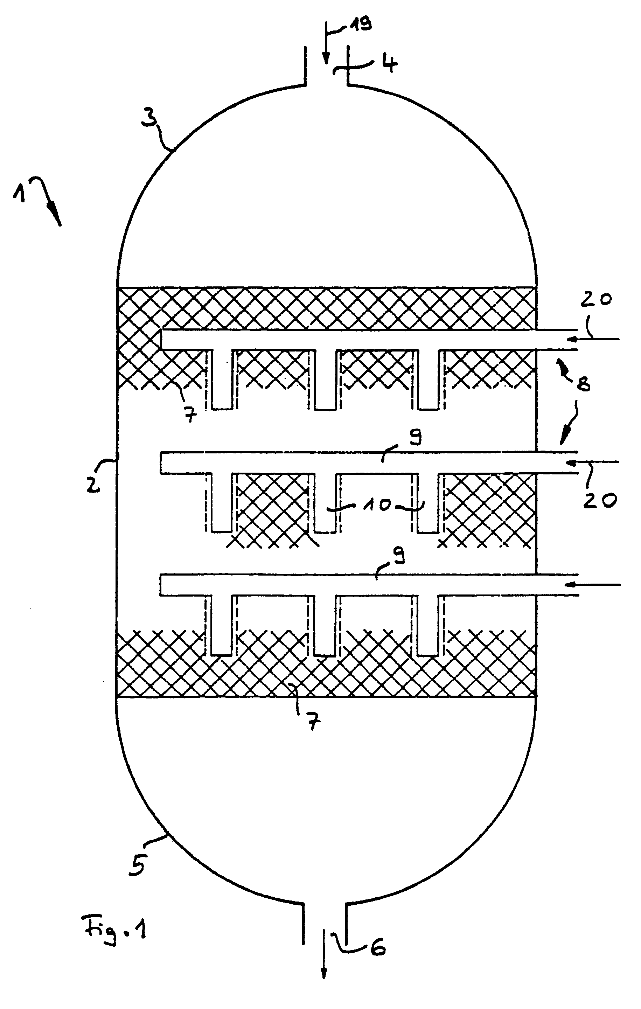

Wie in Fig. 1 dargestellt ist, strömt das eigentliche Reaktionsgas durch den Gaseinlaßstutzen 4 in Richtung des Pfeils 19 ein, der den Hauptgasfluß charakterisiert. Gleichzeitig wird durch die Zentralrohre 11 von außen Kaltgas zugeführt, was durch Pfeile 20 angedeutet ist. Das Reaktionsgas, das sich durch die Reaktion im Katalysatorbett 7 erhitzt, strömt im wesentlichen in der Hauptgasflußrichtung 19 von oben nach unten durch das Katalysatorbett 7, während das Kaltgas durch die Zentralrohre 11 und die Verteilerrohre 12 in die Düsenrohre 10 gelangt und fein verteilt aus den Düsenöffnungen 16 in das Katalysatorbett etwa senkrecht zur Hauptgasrichtung 19 austritt. Dabei wird das Kaltgas derart unter Druck in das Zentralrohr 11 eingeleitet, daß ein gleichmäßiger Austritt aus allen Düsenöffnungen 16 gewährleistet ist.

Das im Katalysatorbett 7 fein verteile Kaltgas vermischt sich mit den heißen Reaktionsgasen, was zu einer Abkühlung der Reaktionsgase führt, wobei durch die Vielzahl der nebeneinander in einer jeweiligen Ebene angeordneten Düsenrohre 10 über dem jeweiligen Reaktorquerschnitt ein gleichmäßiges Temperaturprofil entsteht. Außerdem wird durch die Mehrzahl von über der Höhe des Reaktors 1 angeordneten Kaltgaszuführungen 8 ein Ansteigen der Reaktionstemperaturen weitgehend vermieden, so daß sich vom Reaktoreintritt 4 zum Reaktoraustritt 6 ein relativ gleichmäßiges Temperaturprofil mit geringen Temperaturgradienten einstellt.

Natürlich ist die Erfindung nicht auf die in der Zeichnung dargestellten Ausführungsbeispiele beschränkt. Weitere Ausgestaltungen der Erfindung sind möglich, ohne den Grundgedanken zu verlassen. So ist es auch möglich, die Düsenrohre 10 nach oben gerichtet anzuordnen. Außerdem können die einzelnen Zentralrohre 11 auch an eine gemeinsame, den Reaktormantel durchdringende Kaltgaszuleitung angeschlossen sein und dergl. mehr.

**Patentansprüche**

1. Reaktor (1) zur Durchführung katalytischer Gasreaktionen mit einem Gaseinlaßstutzen (4), im oberen Kugelboden (3) des Reaktors (1) für das gesamte Reaktionsgas mit einer Mehrzahl von über der Höhe des Reaktors angeordneten, in das Katalysatorbett mündenden Kaltgaszuführungen, dadurch gekennzeichnet, daß die Kaltgaszuführungen (8) als senkrecht angeordnete, das Katalysatorbett (7) nur bereichsweise durchdringende Düsenrohre (10) ausgebildet sind, welche mit waagerechten Zuleitungen (9) verbunden sind, wobei die Düsenrohre (10) hängend an den Zuleitungen (9) angeordnet sind, wobei die Düsenrohre (10) jeweils mit einer Abdeckung (15) an ihren stirnseitigen Enden verschlossen sind und wobei die Düsenrohre (10) mit in den Seitenflächen ausgebildeten Düsenöffnungen (16) zum Austritt des eingeleiteten Kaltgases versehen sind.

2. Reaktor nach Anspruch 1, dadurch gekennzeichnet, daß die Zuleitungen (9) jeweils einer Ebene von je einem waagerechten, den Reaktormantel (2) durchdringenden Zentralrohr (11) und beiderseits desselben angeordneten waagerechten Verteilerrohren (12) gebildet sind.

3. Reaktor nach Anspruch 1 oder einem der folgenden, dadurch gekennzeichnet, daß die Verteilerrohre (12) in einem einen Katalysatordurchfluß ermöglichenden Abstand nebeneinander am Zentralrohr (11) angeordnet sind.

4. Reaktor nach Anspruch 1 oder einem der folgenden, dadurch gekennzeichnet, daß die Verteilerrohre (12) mit Verstärkungsrippen (13) versehen sind.

5. Reaktor nach Anspruch 1 oder einem der folgenden, dadurch gekennzeichnet, daß die senkrechten Düsenrohre (10) mit einer perforierten Ummantelung (18) versehen sind.

6. Reaktor nach Anspruch 5, dadurch gekennzeichnet, daß die Ummantelung (18) aus Streckmetall gebildet ist.

**Claims**

1. Reactor (1) for carrying out catalytic gas reactions with a gas inlet nozzle (4) in the upper spherical base (3) of the reactor (1) for the total reaction gas with a plurality of cold gas feeds mounted above the height of the reactor and opening into the catalyst bed, characterised in that the cold gas feeds (8) are formed as vertically mounted nozzle tubes (10) penetrating the catalyst bed (7) only in parts and connected to horizontal feed pipes (9), the nozzle tubes (10) being mounted so as to be suspended from the feed pipes (9), the nozzle tubes (10) each being sealed by a cover (15) at their ends and the nozzle tubes (10) being provided with nozzle apertures (16) in the side faces for discharge of the cold gas introduced.

2. Reactor according to claim 1, characterised in that the feed pipes (9) of each plane are formed of a respective horizontal central pipe (11) penetrating the reactor casing (2) and of horizontal distributing pipes (12) mounted on either side of the central pipe (11).

3. Reactor according to claim 1 or one of the following, characterised in that the distributing pipes (12) are mounted next to one another on the central pipe (11) with a spacing which permits permeation of the catalyst.

4. Reactor according to claim 1 or one of the following, characterised in that the distributing pipes (12) are provided with reinforcing fins (13).

5. Reactor according to claim 1 or one of the following, characterised in that the vertical nozzle tubes (10) are provided with a perforated casing (18).

6. Reactor according to claim 5, characterised in that the casing (18) is formed of rib mesh.

**Revendications**

1. Réacteur (1) pour l'exécution de réactions catalytiques en phase gazeuse, muni d'un raccord (4) d'admission de gaz dans le dôme supérieur (3) du réacteur (1), pour la totalité du gaz de réaction, ainsi que de multiples entrées de gaz froid réparties sur la hauteur du réacteur, et débouchant dans le lit de catalyseur, caractérisé par le fait que les entrées (8) de gaz froid sont réalisées sous la forme de tubes de projection (10) disposés verticalement, qui traversent le lit de catalyseur (7) uniquement par zones et sont reliés à des conduites horizontales d'arrivée (9), les tubes de projection (10) étant suspendus aux conduites d'arrivée (9), ces tubes de projection (10) étant respectivement obturés par un capot (15) à leurs extrémités frontales, et lesdits tubes de projection (10) étant percés d'orifices de projection (16) pratiqués dans les surfaces latérales, en vue de la sortie du gaz froid introduit.

2. Réacteur selon la revendication 1, caractérisé par le fait que les conduites d'arrivée (9) situées dans un plan considéré sont formées, à chaque fois, par un tube central horizontal (11) traversant l'enveloppe (2) du réacteur, et par des tubes répartiteurs horizontaux (12) agencés de part et d'autre dudit tube central.

3. Réacteur selon la revendication 1 ou l'une des suivantes, caractérisé par le fait que les tubes répartiteurs (12) sont agencés en juxtaposition, sur le tube central (11), selon un espacement qui autorise un passage du catalyseur.

4. Réacteur selon la revendication 1 ou l'une des suivantes, caractérisé par le fait que les tubes répartiteurs (12) sont dotés de nervures de renforcement (13).

5. Réacteur selon la revendication 1 ou l'une des suivantes, caractérisé par le fait que les tubes verticaux de projection (10) sont munis d'un chemisage perforé (18).

6. Réacteur selon la revendication 5, caractérisé par le fait que le chemisage (18) est formé d'un métal venu d'étirage.

Fig.1

Fig.3

Fig.2

7

Fig.5

Fig.4